# EUROPEAN PATENT APPLICATION

(11) **EP 1 396 723 A1**
(43) Date of publication of application: **10.03.2004**
(21) Application number: 03445095.7
(22) Date of filing: 14.08.2003
(51) Int. Cl.: G01N 33/34, G01N 7/18, D21C 5/00, D21C 9/10, D21C 9/16

(54) **Method for the measurement of dithionite concentration**

(30) Priority: 04.09.2002 SE 0202615
(71) Applicant: BTG KÄLLE INVENTING AB, S-661 29 Säffle (SE)
(72) Inventor: Bergsland, Helena, 661 31 Säffle (SE); Norder, Stig, 661 33 Säffle (SE)
(74) Representative: Holmberg, Martin

(57) **Abstract**

The concentration of dithionite can be fast and accurately measured in a sample by a method wherein a predetermined amount of peroxide is added to said sample, following which catalase is added to said sample in an incrementally growing dose during a predetermined time, monitoring the production of oxygen in the sample, and recording the onset of oxygen production as well as the amount of oxygen produced as a function of time. The recorded values are then taken as a measure of the amount of dithionite present in the sample.

The above method can be performed in any vessel having an inlet and an outlet, valves for closing these, an agitator, means for adding peroxide and catalase in predetermined amounts, and means for recording the onset of oxygen production, as well as the amount of oxygen produced as a function of time.

## Description

The present invention relates to the field of pulp and paper manufacturing, bleaching methods used within this field, and in particular to a method for measuring the concentration of dithionite, the active compound in dithionite bleaching.

### Background of the invention

One bleaching method for mechanical pulp uses dithionite. Hydrosulfite beaching is another term for dithionite bleaching. The active compound is usually sodium dithionite (Na₂S₂O₄). Dithionite bleaching is frequently used when only a slight increase in pulp brightness is desired, following conventional bleaching steps. Dithionite is a reducing compound, which in solution reacts to produce free sulfite ions, which in turn can oxidize to thiosulfate.

Dithionite is used in particular for the bleaching of recycled fiber pulp, as a reductive bleaching is useful not only for bleaching the pulp itself, but also for destroying dye substances used in coloured paper.

Compared for example to peroxide, the reducing dithionite has a higher kinetic reaction rate that is evident primarily in significantly shorter reaction times. Tests have shown that the bleaching takes only a few minutes. The bleaching effect is positively influenced by rising temperatures and adversely effected by the presence of oxygen. Because of its short reaction time, the homogenous mixing and distribution of dithionite in the pulp is very important.

Under practical operating conditions, the amount of dithionite used in bleaching de-inked pulp is usually 0.5 - 1 % on o.d. pulp. The determination of the true dithionite concentration in the pulp is however very difficult, due to the reactivity of dithionite. Samples must be taken, transported and analysed under the exclusion of oxygen. Under practical conditions, this is often not possible. There is also bound to be at least minute amounts of oxygen present in the pulp itself, which react with the dithionite during the transport to the laboratory, and while awaiting or performing the analysis. Consequently, a reliable determination of the dithionite concentration in the pulp cannot be achieved.

Further, there is a corrosion problem associated with the use of dithionite, due to the presence of thiosulfate in the process fluids, as well as the low pH. Therefore, in order to operate within a margin of safety, dithionite doses added tend to be quite low. Also, dithionite solutions are not very stable, and cannot be stored for long periods, so one batch at the time is prepared, and added to the pulp.

An optimal and efficient use of dithionite would require a simple but reliable method for determining the dithionite concentration in the pulp, preferably on-line.

### Prior art

WO 99/35482 discloses a method and device for measuring the amount or activity of catalase before or in connection with pulp bleaching. A predetermined amount of peroxide is added to a sample inside a closed measurement container, and the generation of oxygen determined. The amount of oxygen then represents a measure of the quantity / activity of catalase in the sample. This method is rapid, simple, and well-suited for performing at location in the pulp and paper mill, and in practice represents an on-line measurement for catalase. A device according to WO 99/35482 consists mainly of a measurement chamber and an adjoining overflow vessel. The sample on which the measurement is to be made enters the container through a first pipe, which can be closed with a first valve. The measurement chamber is connected to the overflow vessel via a second pipe, having a second valve for cutting off the connection between these two volumes. Further, the measurement chamber is provided with an agitator for mixing the contents of said chamber.

When a measurement is to be performed, a predetermined volume (the sample) is trapped in the measurement chamber, a predetermined amount of peroxide added, and the contents of the chamber mixed with an agitator, which initiates the decomposition of the peroxide into water and oxygen. The gas thus formed pushes a corresponding sample volume out of the container and the changed liquid column level is indicated via a pressure sensor.

A device according to WO 99/35482 is commercially available under the trade name CAT 5000 and a corresponding device for the measurement of residual peroxide is available as RPA 5000 (both available from BTG AB, Industrigatan 1-3, 661 29 Säffle, Sweden).

### Summary of the invention

It has surprisingly been found that the concentration of dithionite can be rapidly and accurately measured in a sample by a method wherein a predetermined amount of peroxide is added to said sample, following which catalase is added to said sample in an incrementally growing dose during a predetermined time, and the production of oxygen in the sample monitored, and the onset of oxygen production recorded, as well as the amount of oxygen produced as a function of time. The recorded values are then taken as a measure of the amount of dithionite present in the sample.

The above method can be performed in any vessel having an inlet and an outlet, valves for closing these, an agitator, means for adding peroxide and catalase in predetermined amounts, and means for recording the onset of oxygen production, as well as the amount of oxygen produced as a function of time. A modified device according to WO 99/35482 is suitable for performing the present method, as well the commercially available CAT 5000 and the RPA 5000 device for measuring catalase and residual peroxide, respectively (both from BTG AB, Industrigatan 1-3, 661 29 Säffle, Sweden). None of these is ready for use according to the inventive method, but both require various modifications, which however become evident for the skilled person after reading the present description and claims.

This novel method makes it possible to accurately control the dithionite concentration, and thus to optimise the use of this bleach chemical, improving the bleach result, process economy, and avoiding corrosion problems.

### Short description of the drawings

The invention will be disclosed in closer detail in the description and non-limiting examples, with reference to the attached claims and drawings, in which
Fig. 1 is a diagram showing the onset of oxygen production and amount of oxygen, in a series of tests using clean water only. In this way, a reference curve was obtained.
Fig. 2 is a diagram showing the result from a series of tests, where the dithionite concentration increased from measurement to measurement.
Fig. 3 is a diagram showing the results from a series of tests, using white water from a pulp and paper mill, mixed with buffer filtrate and a known dose of dithionite.

### Description

The present inventors have surprisingly found that it is not necessary to take a sample of a process liquid and subject it to laboratory analysis in order to determine the dithionite concentration is said sample. Instead, the dithionite concentration can be fast and reliably determined practically on line, using a method wherein the following steps are performed:
- a sample of a predetermined volume is taken from a process fluid,
- a predetermined amount of peroxide is added to said sample, in an amount necessary to reach a predetermined level,
- catalase is added to said sample in an incrementally growing dose during a predetermined time,
- the production of oxygen in the sample is monitored,
- the onset of oxygen production as well as the amount of oxygen produced is recorded, and
- the recorded values are taken as a measure of the amount of dithionite present in the sample.

The sample is preferably agitated or stirred during each catalase addition, in order to accelerate the reaction between the dithionite and the catalase. Further, catalase is preferably added incrementally to the sample during a period of 1 to 30 minutes, most preferably during 5 to 15 minutes. It is understood that the rate of catalase addition can be varied, but that it will influence the resolution of the method. In particular, when the catalase is added during a short time period, it may be difficult to accurately determine very low levels of dithionite. A skilled person will however be able to optimise the method and its parameters, such as the rate of catalase addition, taking into account the levels of dithionite normally present in the pulp, as well as the other parameters of the measurement, including the performance and the capacity of the device used.

According to one embodiment, the onset of oxygen production is recorded by a pressure transmitter, for example by trapping the sample in a vessel having a constant volume, leading to a pressure build-up when the oxygen production starts. Another embodiment is to arrange a communicating vessel, into which a part of the sample is forced by the oxygen produced, resulting in a column of liquid corresponding to the amount of oxygen.

This amount of oxygen produced is recorded by a level or pressure transmitter, recording the level of the liquid column in said communicating vessel. Further, the rate of oxygen production is recorded.

The present invention also encompasses a computer program when stored on a carrier, such as a computer disc, a compact disc, a ROM, or operating a processor, said program containing the instructions for performing a method as defmed above. Preferably, said computer program contains an algorithm for correlating the measured parameters to the concentration of dithionite in the sample. Most preferably, said algorithm comprises at least the following variables: the onset of oxygen production, the rate of oxygen production, and the total production of oxygen.

Preferably the algorithm handles also the following variables: the dose of peroxide, the time between the peroxide dose, the catalase dose, and the onset of oxygen production, the initial dose of catalase, and the increasing dose regimen when catalase is added to the sample.

The above method can be performed using a device having the means for performing the functions or process steps defined above. Preferably a modified device according to WO 99/35482 is used for performing the present method, such as the commercially available CAT 5000 and the RPA 5000 device for measuring catalase and residual peroxide, respectively (both from BTG AB, Industrigatan 1-3, 661 29 Säffle, Sweden).

The present invention offers many advantages:

The inventive method is fast and has exhibited good repeatability. The correlation with known dithionite concentrations is good, and the inventive method is assumed to be superior to current laboratory methods, at least when the many sources of error associated with laboratory measurements of dithionite are considered. The inventive method is easily automatised and performed on site in the mill, in practice functioning as an on-line measurement.

By making it possible to accurately measure the concentration of dithionite on-line in the mill, the present invention makes it possible to optimise the use of dithionite. Dithionite can now be adjusted more accurately, at the same time optimising the bleaching and avoiding corrosion. Today the spent bleach liquor continues into the paper mill. It would be a significant advantage if the filtrate, containing dithionite, would be re-circulated in the bleaching, thus preventing the accumulation of catalase and protecting the H₂O₂.

This suggested use of dithionite would also aid in minimizing or avoiding the use of other biocides, as dithionite in combination with H₂O₂ would act as an efficient and environmentally friendly biocide.

### Examples

### 1. Laboratory trials

### 1.1 Dithionite in water

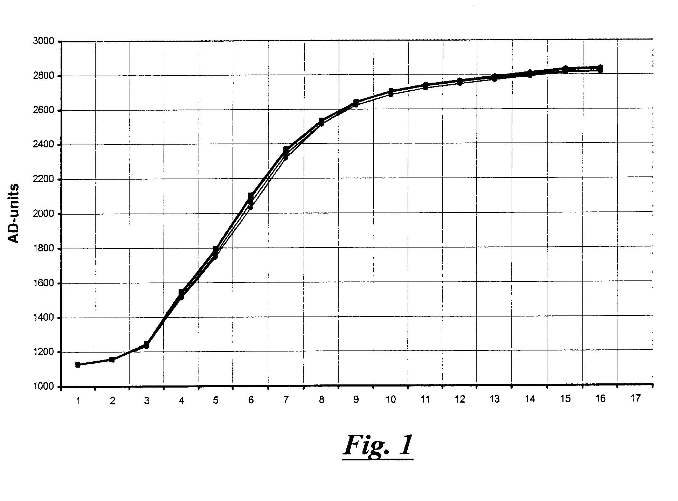

For the laboratory trials, a CAT 5000 measuring device (BTG AB, Industrigatan 1-3, 661 29 Säffle, Sweden) was used. First, a reference was established by performing repeated measurements using clean water, without dithionite. (Fig. 1)

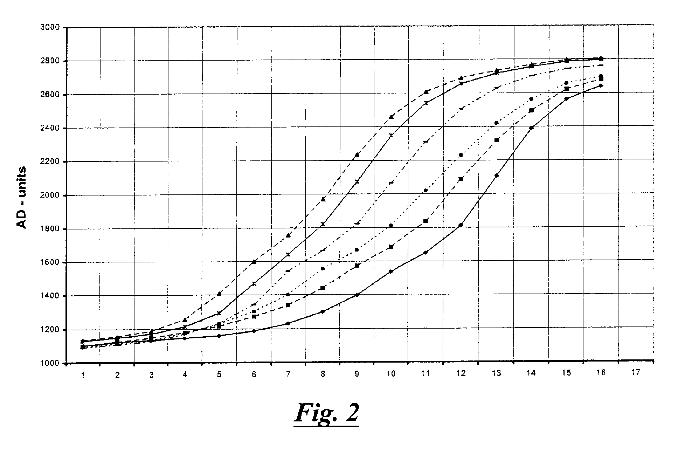

A dithionite solution was then prepared by feeding a total of 30 g dithionite to a 281 vessel initially containing only clean water, and the concentration measured. The dithionite concentration increased slowly until the feed was exhausted, and the concentration started to fall. The results obtained indicate a gradually increasing delay in the onset of oxygen production. The lowest curve (◆) corresponds to the highest dithionite concentration (200 mg/l), and the uppermost curve (▲) corresponds to the lowest concentration (4 mg/l). (Fig. 2)

In both diagrams, the oxygen production is measured in AD-units, representing the pressure increase exerted by the level of liquid in the measurement tube of the CAT 5000 device.

### 1.2 Serial dilution

In this test, the same modified CAT 5000 device was used. 30 g dithionite was added to a vessel containing 28 1 water. When a sample was taken from the vessel for performing a measurement, the vessel was replenished with clean water, keeping a constant volume of 28 l. Thus a serial dilution was automatically achieved. It was shown that the initially very high dithionite concentration influenced the measurement results.

### 1.3 Dithionite feed

The same equipment as above was used, but in this test, a dithionite solution (20 g/l) was fed into the inlet, about 10 cm from the measurement vessel. Nitrogen was bubbled through the dithionite solution in order to guarantee a stable concentration. The results showed a clear dose dependent behaviour.

### 2. Pilot plant trials

Catalase containing filtrates, e.g. the -so-called buffer filtrate, was investigated in a Swedish pulp and paper mill where a considerable amount of recycled paper is used. A portable, modified CAT 5000 device was constructed. The filtrate was first led to a 40 1 vessel, to give the possibility to add dithionate to the filtrate. This vessel was equipped with a stirrer, and for a number of experiments, the headspace of the vessel was filled with nitrogen in order to limit the oxygen decomposition of dithionite.

Peroxide (400 ppm) was added to each sample, followed by catalase solution during five minutes (0.01 ml/ 7.5 sec).

During two days, samples were taken from the buffer tower, via the 40 1 vessel.

During one day, also the white water from a paper machine was investigated.

In both instances, dithionite was added to the filtrate and white water, respectively (27 g; 30 g sodium dithionite, 87 % purity). As the white water contained no catalase at the time of the trials, and the buffer filtrate contained large amounts (100 % decomposition of the peroxide during 15 min), the buffer filtrate was added to the white water in the 40 1 vessel. This mixture turned out to be a suitable sample fluid.

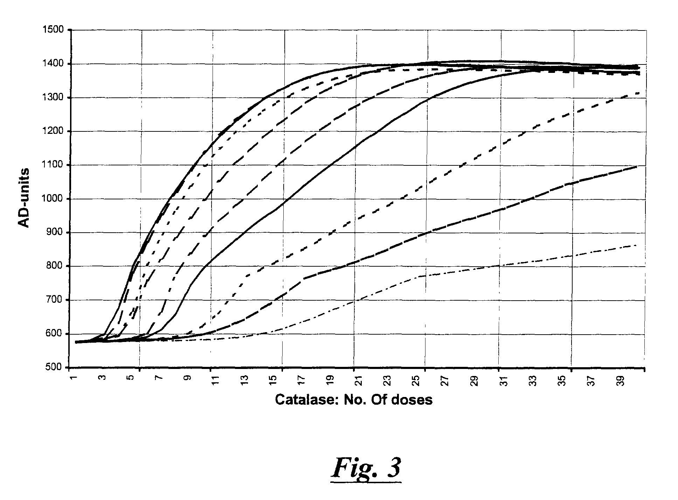

These preliminary studies showed that values, corresponding to the dithionite additions and dilutions performed, were obtained. The lowest curve corresponds to a dithionite concentration of about 200 ppm, and the uppermost curve corresponds to a concentration of about 4 ppm. (Fig. 3)

It only remains to calibrate the equipment for different process fluids, considering that different fluids may contain chemicals that consume catalase, thus influencing the measurements. The experiments performed so far however show that it is possible to obtain an indication of the dithionite concentration in a fast and repeatable way, using an on-line instrument.

Although the invention has been described with regard to its preferred embodiments, which constitute the best mode presently known to the inventors, it should be understood that various changes and modifications as would be obvious to one having the ordinary skill in this art may be made without departing from the scope of the invention as set forth in the claims appended hereto.

## Claims

1. A method for the determination of dithionite concentration in a sample, **characterized in that** the following steps are performed:
- peroxide is added to said sample, in an amount necessary to reach a predetermined level,
- catalase is added to said sample in an incrementally growing dose during a predetermined time,
- the production of oxygen in the sample is monitored,
- the onset of oxygen production as well as the amount of oxygen produced is recorded, and
- the recorded values are taken as a measure of the amount of dithionite present in the sample.

2. The method according to claim 1, **characterized in that** the onset of oxygen production is recorded by a pressure transmitter.

3. The method according to claim 1, **characterized in that** the onset of oxygen production is recorded by a level transmitter.

4. The method according to claim 1, **characterized in that** the amount of oxygen produced is recorded by a pressure transmitter, recording the level of a liquid column in a measurement container.

5. The method according to claim 1, **characterized in that** the rate of catalase addition is recorded.

6. The method according to claim 1, **characterized in that** the rate of oxygen production is recorded.

7. A computer program when stored on a carrier, **characterized in that** said program contains the instructions for performing a method according to claim 1.

8. A computer program according to claim 7, **characterized in that** said program further contains an algorithm for correlating the measured parameters to the concentration of dithionite in the sample.

9. A computer program according to claim 8, **characterized in that** said algorithm comprises at least the following variables: the onset of oxygen production, the rate of oxygen production, and the total production of oxygen.
